# EUROPEAN PATENT APPLICATION

(11) **EP 2 546 329 A1**
(43) Date of publication of application: **16.01.2013**
(21) Application number: 11173463.8
(22) Date of filing: 11.07.2011
(51) Int. Cl.: C12M 3/02

(54) **Scale up of cell cultures**

(71) Applicant: Lonza Cologne GmbH, 50829 Köln (DE)
(72) Inventor: Schenk, Judith, 50678 Köln (DE); van den Bos, Christian, 48268 Greven (DE); Rosenbaum, Claudia, 40591 Düsseldorf (DE); Brill, Silke, 50825 Köln (DE); Safta, Jade, 50823 Köln (DE)
(74) Representative: Remus, Alvaro Johannes

(57) **Abstract**

The invention relates to a method and device for cultivating attachment-dependent cells in the presence of microcarriers in a flexible cultivation container. In particular, the invention concerns a cultivation method using a flexible, scalable, disposable, substantially gas-permeable and non-liquid-permeable container for expansion of adherent cells on microcarriers. According to the invention, cultivation of the cells is accomplished under orbital shaking at 8 rpm or more than 8 rpm.

## Description

### Background of the invention

The invention relates to a method and device for cultivating attachment-dependent cells in the presence of microcarriers in a flexible cultivation container. In particular, the invention concerns a cultivation method using a flexible, scalable, disposable, substantially gas-permeable and non-liquid-permeable container for expansion of adherent cells on microcarriers.

### Prior art

It is well known that cells can be expanded in disposable bioreactors, e.g. plastic bags (Sartorius Stedim Biotech, GE Healthcare). These bioreactors are sterile and ready to use. After use they can be decontaminated and easily disposed. Risk of contamination is lower compared to non-disposable bioreactors and leads to time and cost reduction. Eibel et al. (Appl Microbiol Biotechnol (2010) 86:41-49) presents a good overview of different disposable bioreactor types on the market. However, cell cultures on microcarriers are usually expanded in stirred bioreactors for good oxygen and nutrient transfer. But one problem of microcarrier cell cultures in the stirred system is the balance between high oxygen transfer and shear and collision stress.

US 2011/0070648 A1 discloses the use of a disposable bioreactor for microcarrier cell cultures. Here, cells are cultivated on microcarriers in a plastic bag bioreactor on a rocking device (WAVE system) under hardly noticeable movements. In particular, the cells are initially incubated with the microcarriers in the plastic bag while the bag is kept substantially still so as to allow the cells to adhere to the microcarriers. The cells are then cultivated under constant rocking at less than 7 rpm. However, the ineffective wave mixing of the medium for gas and nutrients by the low rocking movement of the device is a major disadvantage of this method. Thus, this known method is limited by poor mixing and poor mass transfer and therefore only applicable with well established cell cultures (e.g. CHO cells). But even with established cell cultures it is not possible to support high cell densities. It is a further drawback of this method that the cells are cultivated in non-gas-permeable bags and therefore need to be permanently connected to external gassing devices. Keeping the bag at an even temperature is another problem of the described method since the temperature within the bag can only be controlled by heating plates at the bottom of the rocking device.

### Summary of the invention

It is an object of the invention to provide a method and device for cultivating cells on microcarriers in a flexible cultivation container, with which high cell densities can be obtained and cultivation conditions can be controlled easily.

The object is achieved by a method comprising cultivation of cells on microcarriers in a flexible container under orbital shaking at 8 rpm or more than 8 rpm (rpm = rounds per minute), wherein said container comprises a substantially gas-permeable material. According to the invention, the cells are cultured under remarkable agitation on an orbital shaker device so that the cell culture medium is effectively mixed for better transfer of gas and nutrients. Due to the enhanced mixing and mass transfer, higher cell densities and yields can be achieved. Surprisingly, cell damage and mortality rates are still low at the higher shaking rates used in the method according to the invention. As the container is made of or at least comprises a substantially gas-permeable material, permanently connecting the container to external gassing devices is not necessary. Instead, the container can be placed in a suitable incubator providing an atmosphere adapted to the needs of the cells. Moreover, there is no need to provide the container with inlet and outlet members for gas exchange so that the risk of contamination is significantly reduced. Controlling the temperature of the cell culture is also easy within a suitable incubator. In addition, orbital shaking of cells on microcarriers leads to better growth of the cultures and more evenly sized aggregate formation compared to gentle rocking or stationary cultures. Under the conditions according to the invention, there is no foam building and negligible shear stress detectable. Oxygen transfer and mass production for cell expansion is comparable to stirred bioreactors and better than in wave-mixed bioreactors.

According to the invention, bioreactors made of an elastic foil (inflatable bags) are used, which are gas-permeable and non-liquid-permeable. Those bioreactors possess a very high surface for gas exchange with the culture medium compared to non-gas-permeable bags equipped with additional internal gassing valves. The bags can be incubated in every usual incubator under constant temperature and gas conditions (preferably 5% O₂). Orbital shaking of culture bags is easily achieved by placing a usual shaker within a common incubator and does not involve additional heat and gas sources. For removing samples from the culture medium, the container (bioreactor, bag) may comprise a valve or a self-sealing membrane.

According to the invention, commercially available microcarriers, such as Cytodex™ (GE Healthcare, GB), Hillex (SoloHill, USA) or the like, may be used, including but not limited to temperature-controlled microcarriers from which cells are removed by a change of temperature, preferably by lowering the temperature.

In a preferred embodiment of the method according to the invention, the orbital shaking is accomplished at 8-80 rpm, preferably 8-70, 8-60, 8-50, 8-40, 8-30 or 8-20 rpm, further preferred 20-80, 30-80, 40-80, 50-80, 60-80 or 70-80 rpm, also preferred 10-70, 20-60, 30-50, 35-45 or 30-40 rpm. Within these preferred shaking rate ranges mixing of the culture medium, and thus gas transfer, is very efficient so that high cell densities can be achieved while shear and collision stress is still low enough to avoid cell damage and high mortality rates. Moreover, it is easy to adjust the shaking rate within a given range in order to achieve a desired dissolved oxygen concentration on the one hand and to reduce foaming of the medium on the other hand.

In order to allow the cells to adhere to the microcarriers, the container is initially shaken at less than 8 rpm. Surprisingly, there is no need to keep the culture substantially still during the attachment stage. In contrast, shaking of the culture during this stage at low shaking rates is beneficial since sedimentation of the cells is avoided and the likelihood of cell/microcarrier contacts is increased. Preferably, shaking of the cell culture is accomplished initially at 1-7 rpm and subsequently at 8-80 rpm, preferably 8-70, 8-60, 8-50, 8-40, 8-30 or 8-20 rpm, further preferred 20-80, 30-80, 40-80, 50-80, 60-80 or 70-80 rpm, also preferred 10-70, 20-60, 30-50, 35-45 or 30-40 rpm. The initial shaking may have a duration of up to 12 h, preferably 1-6 h, further preferred 1-4, in particular 1-2 h.

According to the invention, shaking can be accomplished constantly or intermittently. If intermittent shaking is used, the shaking rate is preferably varied between a lower rate in the range of 8-20 rpm and a higher rate in the range of 20-40 rpm. However, the mode and rate of shaking may be adapted to the cell type to be cultured and the other culturing conditions.

According to preferred embodiment of the method according to the invention, at least the inner surface of said container comprises a non-stick material so as to avoid undesired attachment of cells and microcarriers to the inner surface of the container.

In another preferred embodiment of the invention, the container is made of a non-stick material or at least in part coated with a non-stick material in order to avoid undesired attachment of cells and microcarriers to the surface of the container.

In another preferred embodiment of the invention, said non-stick material comprises polytetrafluoroethylene (PTFE, trade name (DuPont): Teflon^{®}). Bioreactors made of PTFE foil are gas permeable and non-liquid permeable. Bioreactors made of PTFE or coated with PTFE possess a very high surface for gas exchange with the culture medium compared to non-gas permeable bags equipped with additional internal gassing valves. Further, the PTFE surface of the container effectively prevents cells and microcarriers from adhering to the inner side of the container. However, any other biocompatible, non-stick material may be used for producing or coating the container, for example, polyfluoroethylene (PFE), perfluoroalkoxy (PFA), fluorinated ethylene propylene (FEP), a titanium dioxide compound or a titanium dioxide comprising composition.

For cell expansion additional microcarriers may be added to the existing culture so as to provide additional attachment area for the increasing cell number. Thus, expansion of cell cultures is easily achieved by mere addition of more microcarriers. For certain applications it may be beneficial to use differently sized microcarriers.

The object is further achieved by a device for cultivating attachment-dependent cells in the presence of microcarriers in a flexible cultivation container, said device comprising at least one shaking device comprising at least one means for holding the container and being capable of orbitally moving the container.

Optionally, the device may comprise a control unit for controlling the shaking device so as to allow for automatic intermittent shaking.

The object is further achieved by an arrangement for cultivating attachment-dependent cells in the presence of microcarriers in a flexible cultivation container, comprising:
- At least one chamber comprising at least one means for holding the container;
- At least one shaking device comprising said means for holding the container and being capable of orbitally moving the container;
- At least one temperature control system for controlling the temperature within said chamber; and
- At least one gassing system for controlling the concentration of at least one gas within said chamber.

Using the device and/or arrangement according to the invention allows for easy and more effective expansion of cells because culture conditions can be easily and exactly adjusted. As the container can be or is placed within a chamber having a controlled atmosphere, permanently connecting the container to external gassing devices is not necessary. Additionally, avoiding inlet and outlet members for gas exchange significantly reduces the risk of cell culture contamination. The temperature of the cell culture is controlled by the temperature control system. In addition, as the container can be placed on a shaking device providing sufficient agitation, orbital shaking of the cells on microcarriers leading to better growth of the cells compared to rocking or stationary cultures is easily accomplished.

More closely, the present invention relates to a method and device for expansion of cells, such as mesenchymal stem cells, on microcarriers in a plastic bag bioreactor. The invention enables expansion of stem cells to therapeutic amounts. The method according to the invention preferably comprises the following steps:
a) Addition of cells and microcarriers in a suitable cell culture medium to a flexible, gas-permeable container,
b) allowing the cells to adhere to the microcarriers while the container is orbitally shaken at less than 8 rpm, this step being preferably accomplished in a small (reduced) volume so as to enhance the likelihood of cell-microcarrier-contacts.
c) optionally adding further cell culture medium if the foregoing step was accomplished in a small (reduced) volume,
d) culturing the cells under orbital shaking at 8 rpm or more than 8 rpm,
e) optionally adding further microcarriers for expansion, and
f) harvesting of cells by enzymatic detachment or detachment from temperature-controlled microcarriers by a change of temperature, preferably by lowering the temperature.

Expansion of cells can be accomplished in batch modus or perfusion modus.

The main advantages of the method and device according to the invention are as set forth hereunder:
- High cell densities;
- High yield;
- Improved dissolved oxygen in cell culture;
- Easy control of conditions, e.g. dissolved oxygen concentration and/or temperature;
- Production of uniformly sized cell aggregates that are able to proliferate;
- Production of inoculum and large scale production within the same cultivation system (no change of inoculum pre-treatment compared to mass production scale);

The invention is further exemplarily described in detail with reference to the figures.

### Brief description of the figures

Figure 1 shows a bar diagram of the total yield of cells per culture bag expanded by the method according to the invention compared to the yield of cells expanded by a prior art method;
Figure 2 shows another diagram of the total yield of cells per culture bag expanded by the method according to the invention compared to the yield of cells expanded by a prior art method for 300 ml and 550 ml volume;
Figure 3 shows a microscopic view on a cell culture of PRAD cells expanded on microcarriers using the WAVE bioreactor after 5 days of culturing, rocking rate: 8 rpm, rocking angle: 2°;
Figure 4 shows a microscopic view on a cell culture of PRAD cells expanded on microcarriers using the WAVE bioreactor after 16 days of culturing, rocking rate: 8 rpm, rocking angle: 2°;
Figure 5 shows a microscopic view on a stationary cell culture of PRAD cells expanded on microcarriers after 5 days of culturing;
Figure 6 shows a microscopic view on a stationary cell culture of PRAD cells expanded on microcarriers after 16 days of culturing;
Figure 7 shows a microscopic view on a cell culture of PRAD cells expanded on microcarriers using the method according to the invention after 5 days of culturing, shaking rate: 24 rpm; and
Figure 8 shows a microscopic view on a cell culture of PRAD cells expanded on microcarriers using the method according to the invention after 16 days of culturing, shaking rate: 24 rpm.

### Example

### Proliferation of PRAD cells on microcarriers in a flexible bag in stationary, rocking and shaking mode:

Since PRAD cells showed a high proliferation rate and strong clumping of beads when cultured in a Lumox foil bag (see SBI_110110 WaveBR with PRADs), the proliferation of PRAD cells on Hillex II microcarriers in a Lumox foil bag in stationary, rocking and shaking mode was tested.
- Media/Materials:
   o EBM-2 basal media+ EGM singlequots (Lonza)
   o Hillex II (Solohill; H112-170515 cm2/ 1g → 5g beads)
   o Pluronic F-168 (P1300 Sigma; 100x in EGM-2; working conc. 0,5g/L)
   o Lumox foil bag
   ο 3x 500mL shake flasks, silanized
- Instruments:
   o ChemoMetec NucleoCounter
   o WAVE Bioreactor 2/10 (GE Healthcare)
   o Shaker base
- Cells:
   o K.LA.PRAD22.Feb10 p1, directly thawed
- Experimental Setup:
   *o Condition:* Pre-seed cells in three 500mL shake flasks: 10x10E6 K.LA.PRAD22.Feb10 p2, thawed as p1, counted and then added to 3,25g Hillex II beads and 200mL EGM-2 media in each shake flask, stationary for 24 hours (seeded: ca. 5974 cells/cm2).
- Day 0:
   1. Fill three 500mL shake flasks with 200mL EGM-2, 0,5g/L Pluronic and 3,25g Hillex II each. Add 10x10E6 PRAD cells drop wise to each flask, shake a bit and incubate them stationary 24h.
- Day 1:
   1. Fill three Lumox bags with the attached PRADs on Hillex II. Add 100mL fresh media (300mL in total). Incubate the bags stationary overnight.
- Day 2:
   1. Count cells.
   2. Start rocking one bag on the Wave at 2°, 8rpm.
   3. Start shaking one bag on the shaker base at 24rpm.
   4. One bag stays stationary.
- Day 5:
   1. Count cells.
- Day 7:
   1. Count cells.
   2. Add 1,25g beads and 150mL media.
- Day 9:
   1. Count cells.
- Day 12 (14.02):
   1. Measure glucose, lactate and count cells.
   2. Add 1g beads and 100mL media, increase rpm to 30rpm on the shaker.
- Day 14:
   1. Count cells.
- Day 16:
   1. Count cells.
   2. Add 1,25g beads and 150mL media
   3. Stop experiment

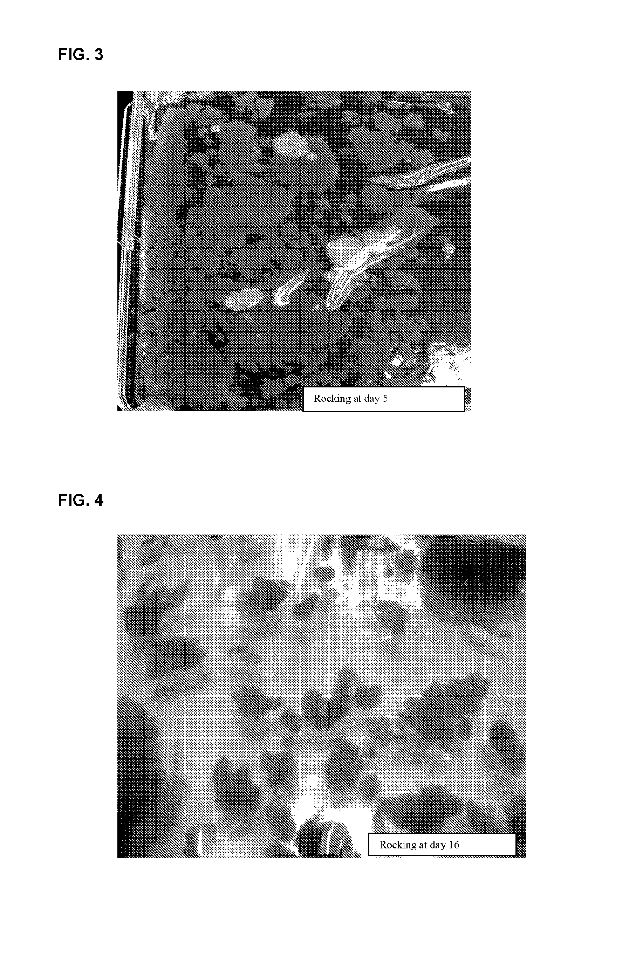

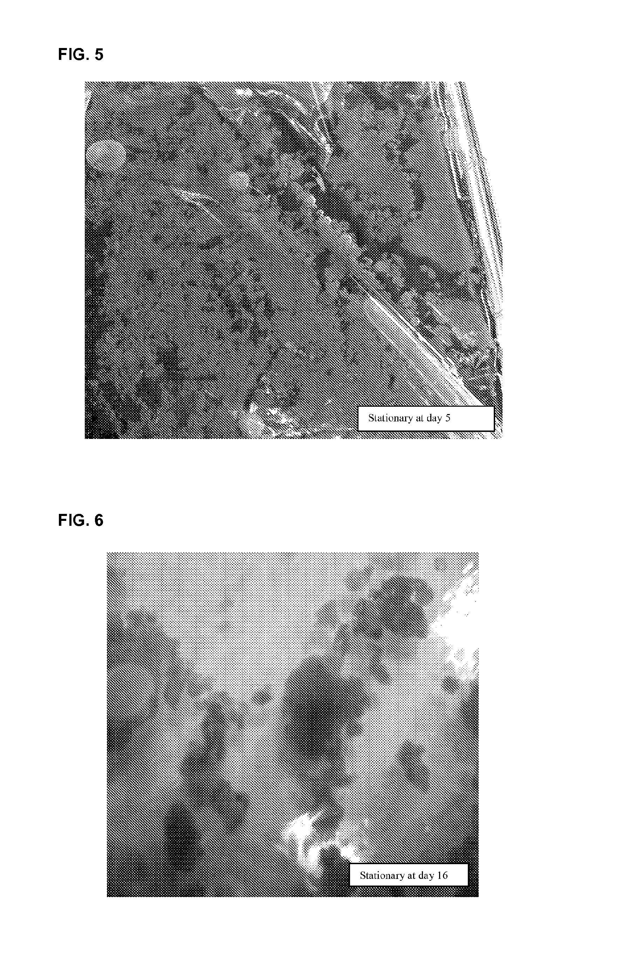

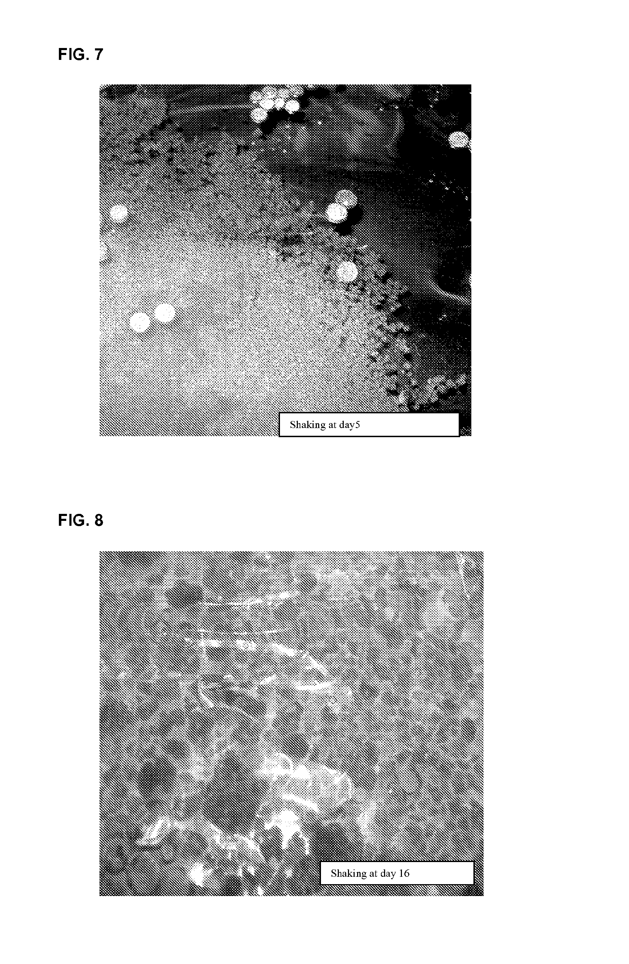

Day 5: Aggregation of beads: The rocked beads formed asymmetric aggregates, very small ones and bigger ones, some of them were flat, some not (**Fig. 3**). The beads in the stationary condition formed a monolayer-like flat shape (**Fig. 5**). Beads formed uniform-shaped aggregates in the shaking conditions, small ball-like aggregates (**Fig. 7**).

Day 11: Bead addition: Three days after addition of beads, the microcarriers are more evenly populated in the shaking condition, compared to the other two conditions. This is due to the fact that the microcarriers are evenly distributed in the medium because of orbital shaking. Contrary, in the rocking and especially in the stationary condition some beads were very dense populated and some beads were empty.

Day 16: Aggregation of beads: The rocked beads formed asymmetric aggregates, mid-sized ones and bigger ones (**Fig. 4**). The beads in the stationary condition also formed asymmetric aggregates, mid-sized ones and bigger ones (**Fig. 6**). Beads now formed bigger, uniform-shaped aggregates in the shaking conditions, smaller, ball-like aggregates (**Fig. 8**).

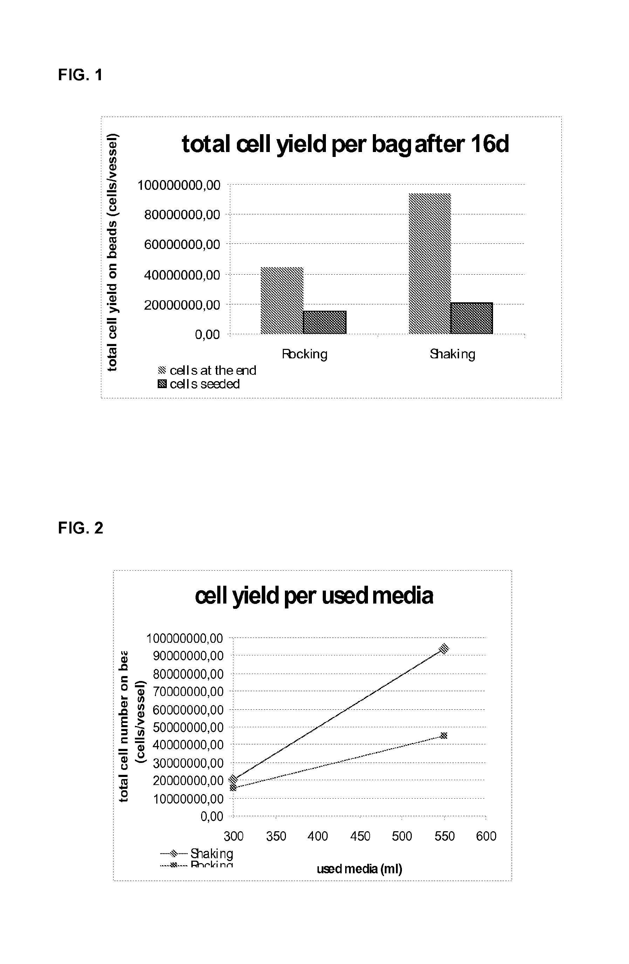

**Figure 1** shows a comparison between a prior art method for cell expansion using a WAVE rocking device ("Rocking") and the method according to the invention ("Shaking"). Here, it is demonstrated that cell expansion according to the invention (orbital shaking at 24 rpm) results in a much higher cell yield than a method using a conventional WAVE bioreactor (rocking at 8 rpm).

**Figure 2** demonstrates that scale up of cell cultures is improved by the method according to the invention ("Shaking") since the difference in cell yield between the method according to the invention and the WAVE rocking method ("Rocking") increases with increasing volume. In the larger scale (550 ml), cell expansion according to the invention (orbital shaking at 24 rpm) results in a much higher cell yield than the conventional WAVE rocking method (rocking at 8 rpm).

## Claims

1. Method for cultivating attachment-dependent cells in the presence of microcarriers in a flexible cultivation container, said method comprising cultivation of the cells under orbital shaking at 8 rpm or more than 8 rpm, wherein the container comprises a substantially gas-permeable material.

2. Method according to claim 1, wherein said orbital shaking is accomplished at 8-80 rpm, preferably 8-70, 8-60, 8-50, 8-40, 8-30 or 8-20 rpm, further preferred 20-80, 30-80, 40-80, 50-80, 60-80 or 70-80 rpm, also preferred 10-70, 20-60, 30-50, 35-45 or 30-40 rpm.

3. Method according to claim 1 or 2, wherein said container is initially shaken at less than 8 rpm in order to allow the cells to adhere to the microcarriers.

4. Method according to claim 3, wherein shaking is accomplished initially at 1-7 rpm and subsequently at 8-80 rpm, preferably 8-70, 8-60, 8-50, 8-40, 8-30 or 8-20 rpm, further preferred 20-80, 30-80, 40-80, 50-80, 60-80 or 70-80 rpm, also preferred 10-70, 20-60, 30-50, 35-45 or 30-40 rpm.

5. Method according to claim 3 or 4, wherein said initial shaking has a duration of up to 12 h, preferably 1-6 h, further preferred 1-4, in particular 1-2 h.

6. Method according to any one of claims 1 to 5, wherein orbital shaking is accomplished intermittently, preferably varied between a lower rate in the range of 8-20 rpm and a higher rate in the range of 20-40 rpm.

7. Method according to any one of claims 1 to 6, wherein at least the inner surface of said container comprises a non-stick material.

8. Method according to any one of claims 1 to 7, wherein said container is made of a non-stick material or wherein said container is at least in part coated with a non-stick material.

9. Method according to claim 7 or 8, wherein said non-stick material comprises polytetrafluoroethylene (PTFE).

10. Method according to any one of claims 1 to 9, wherein additional microcarriers are added to the existing culture for cell expansion.

11. Device for cultivating attachment-dependent cells in the presence of microcarriers in a flexible cultivation container, said device comprising at least one shaking device comprising at least one means for holding the container and being capable of orbitally moving the container.

12. Device according to claim 11 comprising a control unit for controlling the shaking device so as to allow for automatic intermittent shaking.

13. Arrangement for cultivating attachment-dependent cells in the presence of microcarriers in a flexible cultivation container, comprising:
- At least one chamber comprising at least one means for holding the container;
- At least one shaking device comprising said means for holding the container and being capable of orbitally moving the container;
- At least one temperature control system for controlling the temperature within said chamber; and
- At least one gassing system for controlling the concentration of at least one gas within said chamber.
